# EUROPEAN PATENT SPECIFICATION

(11) **EP 0 265 137 B1**
(45) Date of publication and mention of the grant of the patent: **22.12.1993**
(21) Application number: 87309000.5
(22) Date of filing: 12.10.1987
(51) Int. Cl.: C07C 51/487, C07C 63/26, C07C 51/215

(54) **Purifying terephthalic acid**
Reinigung von Terephthalsäure
Epuration de l'acide téréphtalique

(30) Priority: 20.10.1986 JP 247500/86
(43) Date of publication of application: 27.04.1988
(73) Proprietor: MITSUI PETROCHEMICAL INDUSTRIES, LTD., Tokyo 100 (JP)
(72) Inventor: Shiraki, Shigemi, Iwakuni-shi Yamaguchi (JP); Mizuno, Kenichi, Iwakuni-shi Yamaguchi (JP)
(74) Representative: Perry, Robert Edward

(56) References cited:
- EP-A- 0 083 224
- US-A- 3 584 039
- US-A- 4 476 242
- PATENT ABSTRACTS OF JAPAN, vol. 5, no. 148 (C-72)(820), 18th September 1981; & JP-A-5679635
- PATENT ABSTRACTS OF JAPAN, vol. 2, no. 24 (4119C77), 16th February 1978; & JP-A-52122333

## Description

### Field of the Invention

The present invention relates to a process for the production of high purity terephthalic acid.

### Background of the Invention and Prior Art

Terephthalic acid is obtained by oxidising a p-dialkylbenzene such as p-xylene. The oxidation reaction is usually performed in a solvent such as acetic acid, propionic acid, butyric acid, isobutyric acid, valeric acid, trimethylacetic acid, caproic acid or a mixture thereof with water, using an oxygen-containing gas such as air, in the presence of a heavy metal and/or bromine-containing catalyst, under high pressure, in the liquid phase, at high temperature.

By such an oxidation reaction using, for example, p-xylene as the starting material, crude terephthalic acid is formed via p-toluic acid and 4-CBA (4-carboxybenzaldehyde). In the reaction mixture, the terephthalic acid which is formed precipitates as crude terephthalic acid crystals including impurities such as 4-CBA in the solvent. The crude terephthalic acid product usually includes many impurities in addition to 4-CBA.

On account of the impurities, the crude product is unsuitable for the production of polymer-grade polyesters by direct reaction with a glycol; for example, unacceptable coloration results. The production of terephthalic acid is usually accompanied by mild oxidation, in order to decrease loss of the solvent (such as acetic acid), whereby the amount of impurities is further increased.

Various procedures have been proposed, for obtaining high purity terephthalic acid by purifying terephthalic acid containing impurities. Japanese Patent Publications Nos. 20820/1966, 23447/1968 and 23448/1968 disclose oxidation or hydrogenation of the crude terephthalic acid in suspension in water or a water/acetic acid solvent. Proposals based on treatment by oxidation or reduction of an aqueous or water/acetic acid solution of crude terephthalic acid, which brings about higher catalytic efficiency than the given technique of treating the crude terephthalic acid in suspension, are found in Japanese Patent Publications Nos. 21819/1967, 16860/1966, 46212/1977, 10051/1978, 32319/1981, 35174/1981, 35653/1981, 51373/1982, 51374/1982 and 51818/1982, and Kokai 1369/1973, 79635/1981 and 103136/1981.

Post-oxidation of the reaction mixture, by heating it directly after the reaction, is disclosed in Japanese Patent Publication No. 12695/1965. Treatment of an aqueous solution of crude terephthalic acid with a catalyst of palladium or zinc is disclosed in Japanese Patent Publications Nos. 29131/1971, 3607/1972, 13780/1974 and 33189/1974. Treatment of an aqueous alkaline solution of crude terephthalic acid with oxygen is described in Kokai 113738/1981.

All the given techniques for purifying crude terephthalic acid are effective to some degree, when the content of impurities is relatively low, but they are not always satisfactory if the impurity content is high. In many cases, the colour of the crude terephthalic acid is not improved, although the content of 4-CBA which is the principal impurity can be reduced. In the prior art processes, there is little or no conversion of 4-CBA into terephthalic acid; most 4-CBA therefore goes to waste, so that there is no improvement in the yield of terephthalic acid and the amount of residue resulting from the purification process increases.

For example, Japanese Patent Publication No. 46212/1977 describes that a 4-CBA content of 2,300 ppm can be reduced to 0 ppm by a method in which a high temperature aqueous solution of crude terephthalic acid is treated in the presence of a catalyst with a gas mixture containing oxygen, and the terephthalic acid is crystallised. Most of the 4-CBA is converted, not into terephthalic acid but into a waste product in the form of benzoic acid (by decarboxylation). Therefore, there is no increase in the yield of terephthalic acid, for an increased 4-CBA content. Moreover, the colour of the purified terephthalic acid is not satisfactory. The substance causing discoloration cannot be removed sufficiently by the oxidation treatment; indeed, the amount of that substance may even increase under the conditions.

Again, the purification method based on the reduction of an aqueous solution of crude terephthalic acid with hydrogen, as proposed in Japanese Patent Publication No. 16860/1966, gives no increase in the yield of terephthalic acid, since in this case 4-CBA is converted into p-toluic acid.

### Summary of the Invention

A novel process for purifying terephthalic acid comprises oxidising an aqueous solution of crude terephthalic acid containing 4-carboxybenzaldehyde by reaction with 0.4-10 moles oxygen per mole of the 4-carboxybenzaldehyde; and treating the oxidised system with hydrogen.

### Detailed Description of the Invention

Crude terephthalic acid to be purified in the process according to the present invention is as obtained in the process described above. It may contain at least 1,000 ppm, and preferably more than 2,000 ppm, 4-CBA. Less stringent oxidation conditions are required for a higher content of 4-CBA, and loss of the reaction medium on oxidation can be therefore minimised. By contrast to the prior art, the novel process oxidises 4-CBA to terephthalic acid (without decarboxylation), thus giving a high yield of terephthalic acid.

For the first step of the novel process, crude terephthalic acid is dissolved in water, usually under pressure, with heating. While it is preferable to employ pure water, a small amount of the reaction medium used in the production of the crude terephthalic acid, such as acetic acid, may be included.

For the oxidation treatment, an oxygen-containing gas such as air, optionally together with an inert gas such as nitrogen, is used. The oxygen feed rate should be chosen at a value sufficient for the oxidation of 4-CBA but as low as is possible in order to conduct the succeeding reduction treatment advantageously. The feed rate of oxygen into the oxidation treatment system is 0.4-10 moles, preferably 0.5-5 moles, of oxygen per mole of 4-CBA contained in the crude terephthalic acid. If the oxygen feed rate is lower than the given lower limit, 4-CBA tends to be converted into benzoic acid by decarboxylation; within the given feed rate range, almost all the 4-CBA is oxidized into terephthalic acid; if the oxygen feed rate exceeds the given upper limit, oxidation of terephthalic acid occurs with the results that the yield is reduced and that coloured impurities (which cannot be removed by the subsequent hydrogen treatment) are produced.

In the oxidation treatment a catalyst such as activated carbon or a copper-containing, cobalt-containing or molybdenum-containing carrier catalyst can be employed in order to promote the reaction. Particular examples of the last three catalysts are oxides of copper, cobalt or molybdenum on a carrier such as Al₂0₃. It is also possible to use a mixed metal oxide catalyst, e.g. the oxides of metals such as copper/zinc or cobalt/molybdenum. When using a catalyst, a fixed bed reactor is employed. Depending on the purity of the crude terephthalic acid product, the conditions for the oxidation treatment are usually, for a concentration of the crude terephthalic acid in water of 100 to 700 g/l, a temperature of 230 to 300°C, a pressure of 30 to 100 Kg/cm² and a residence time of 2 to 50 minutes.

The hue or colour of the crude product caused by impurities is not improved by the oxidation treatment, and may, in some cases, deteriorate. The colour can be improved by the subsequent hydrogen treatment.

The high temperature aqueous solution of crude terephthalic acid which has been subjected to the oxidation treatment is treated with hydrogen, preferably after passing through a deaeration vessel or an oxygen-absorbing layer to remove oxygen.

For the hydrogen treatment, a conventional technique employed for the purification of crude terephthalic acid may be applied. The conditions are preferably a temperature of 270-300°C, a hydrogen partial pressure of 5-15 Kg/cm², and a treatment time of 2 to 50 minutes. The catalyst may be any of those given in JP-B-16860/1966, e.g. palladium, ruthenium, rhodium, osmium, iridium, platinum, platinum black, palladium black or iron-cobalt-nickel, e.g. on an activated carbon carrier.

After the hydrogen treatment, a high purity terephthalic acid product exhibiting superior hue can be obtained by allowing precipitation of terephthalic acid, followed by conventional procedures such as filtration and centrifugation. If desired, further procedures such as treatment with activated carbon and/or washing with water may be used.

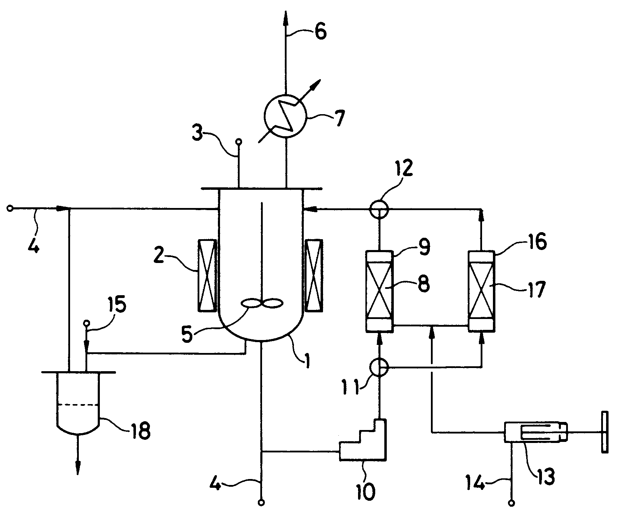

The following Examples illustrate the invention. The apparatus shown schematically in the accompanying drawing was used (some valves, e.g. pressure control valves, are not shown). Solution temperature was accurately controlled by an electric heater (not shown) in combination with heat insulation, in order to prevent blockage of lines.

In the Examples, the content of 4-CBA was determined by polarographic analysis; the light transmittance for each terephthalic acid product is given as T₄₀₀ which is a percentage transmittance for light of 400 mµ passed through a layer of a 2 N aqueous potassium hydroxide solution containing 15% w/w terephthalic acid (this is also called an alkaline transmittance).

### Example 1

Gas in the apparatus was first replaced by nitrogen. Then, 100 g of a crude terephthalic acid having a 4-CBA content of 5,100 ppm and T₄₀₀ = 52% were charged into a 2 litre vessel 1 equipped with a heater 2 through a raw material supply line 3, whereupon the entire system was pressurised to a pressure of 80 Kg/cm² gauge. Thereafter, 400 ml hot water were introduced via hot water inlets 4, and the temperature of the mixture was elevated to 285°C by the heater 2, with agitation. The agitation was continued by means of an agitator 5 for 30 minutes. The internal pressure of the slurry dissolution vessel 1 was controlled so as to maintain the pressure of 80 Kg/cm² gauge by constantly discharging the gas evolved to the outside of the reaction system via a vent line 6 after passage through a cooler 7.

The content of the vessel 1 was circulated through an oxidising vessel 9 having an internal volume of 80 ml charged by an activated carbon layer 8 via a pump 10 and two-way valves 11 and 12, while continually introducing air from an air/hydrogen inlet 14 by means of an injection pump 13. The oxidation treatment under circulation of the solution through the oxidising vessel 9 was performed at a constant circulation rate of 5 litres/h for 60 minutes, during which the total amount of air fed was 0.29 N litre.

Thereafter, the air dissolved in the solution was purged by introducing nitrogen gas from line 15 over 10 minutes.

The two-way valves 11 and 12 were then actuated and the solution was treated with hydrogen by being circulated through a hydrogen treatment vessel 16 at the same temperature. The hydrogen treatment vessel 16 contained a layer 17 of 80 ml palladium-on-carbon catalyst. A total amount of 0.40 N litre hydrogen gas was fed in intermittently from the air/hydrogen inlet 14 by means of the injection pump 13. The duration of hydrogen circulation was 30 minutes.

Subsequently the system was cooled to 130°C and the internal pressure was released, whereupon the contents were subjected to a pressure filtration through a filter 18 and then to washing with hot water.

The properties of the terephthalic acid obtained from the filter 18 were determined. The 4-CBA content was 5 ppm; T₄₀₀ = 98%. The total amount of 4-CBA and p-toluic acid in the filtrate was 0.02 g.

### Example 2

The procedure of Example 1 was repeated seven times without replacing the catalysts in the hydrogen treatment vessel 16 and in the oxidation vessel 9. There was almost no change in the material properties of the product obtained.

### Comparative Example 1

The procedure of Example 1 was repeated, except that the reaction mixture was cooled to 130°C directly after the oxidation treatment and was subjected to a pressure filtration; the hydrogen treatment was omitted. The catalysts in the oxidation vessel 9 and in the hydrogen treatment vessel 16 were each replaced by new ones.

The 4-CBA content of the terephthalic acid product was 100 ppm; T₄₀₀ decreased to 30 %.

### Comparative Example 2

The hydrogen treatment procedure of Example 1 was repeated, without the oxidation treatment. The 4-CBA content of the terephthalic acid product obtained was found to be 15 ppm; T₄₀₀ = 98%. The total amount of 4-CBA and p-toluic acid was 0.47 g.

### Comparative Example 3

The procedure of Comparative Example 2 was repeated five times. After the fifth repetition, T₄₀₀ had decreased steeply, to 81%.

## Claims

1. A process for purifying terephthalic acid, which comprises oxidising an aqueous solution comprising 100 to 700 g/l of crude terephthalic acid containing, with respect to the acid, at least 1000 ppm 4-carboxybenzaldehyde, by reaction with 0.4-10 moles oxygen per mole of the 4-carboxybenzaldehyde in the presence of a catalyst, in a fixed bed reactor; and treating the oxidised system with hydrogen.

2. A process according to claim 1, wherein the oxidation is carried out in an oxidation vessel at 230-300°C under a pressure of 30-100 Kg/cm² and over a residence time of the solution in the oxidation vessel of 2-50 minutes.

3. A process according to either preceding claim, wherein the hydrogen treatment is carried out at 270-300°C under a hydrogen partial pressure of 5-15 Kg/cm² over a period of 2-50 minutes.

4. A process according to any preceding claim, which comprises the addition steps of preparing the aqueous solution by oxidising a 1,4-dialkylbenzene, and dissolving the resultant crude terephthalic acid in water.

## Patentansprüche

1. Verfahren zur Reinigung von Terephthalsäure, umfassend die Oxidation einer wäßrigen Lösung, die 100 bis 700 g/l Roh-Terephthalsäure, die bezogen auf die Säure wenigstens 1000 ppm 4-Carboxybenzaldehyd enthält, umfaßt, durch Umsetzung mit 0,4-10 Mol Sauerstoff pro Mol des 4-Carboxybenzaldehyds in der Anwesenheit eines Katalysators in einem Festbett-Reaktor; und die Behandlung des oxidierten Systems mit Wasserstoff.

2. Verfahren nach Anspruch 1, in welchem die Oxidation in einem Oxidationsgefäß bei 230-300°C und einem Druck von 30-100 kg/cm² und über eine Verweilzeit der Lösung im Oxidationsgefäß von 2-50 Minuten hinweg durchgeführt wird.

3. Verfahren nach irgendeinem der vorangehenden Ansprüche, in welchem die Wasserstoff-Behandlung bei 270-300°C unter einem Wasserstoff-Partialdruck von 5-15 kg/cm² über eine Zeitspanne von 2-50 Minuten hinweg durchgeführt wird.

4. Verfahren nach irgendeinem vorangehenden Anspruch, welches die Zusatz-Schritte der Herstellung der wäßrigen Lösung durch Oxidation eines 1,4-Dialkylbenzols und der Auflösung der resultierenden Roh-Terephthalsäure in Wasser umfaßt.

## Revendications

1. Procédé de purification d'acide téréphtalique qui comprend l'oxydation d'une solution aqueuse contenant de 100 à 700 g/l d'acide téréphtalique brut contenant, par rapport à l'acide, au moins 1000 ppm de 4-carboxybenzaldéhyde, par réaction avec 0,4 à 10 mol d'oxygène par mole du 4-carboxybenzaldéhyde en présence d'un catalyseur, dans un réacteur à lit fixe ; et le traitement du système oxydé par de l'hydrogène.

2. Procédé selon la revendication 1, dans lequel l'oxydation est mise en oeuvre dans un récipient d'oxydation à 230 à 300°C sous une pression de 30 à 100 kg/cm² et avec un temps de séjour de la solution dans le récipient d'oxydation de 2 à 50 min.

3. Procédé selon l'une ou l'autre des revendications précédentes, dans lequel le traitement par l'hydrogène est mis en oeuvre à 270 à 300°C sous une pression partielle d'hydrogène de 5 à 15 kg/cm² pendant une période de 2 à 50 min.

4. Procédé selon l'une quelconque des revendications précédentes qui comprend les étapes additionnelles de préparation de la solution aqueuse par oxydation d'un 1,4-dialkylbenzène et dissolution de l'acide téréphtalique brut résultant dans de l'eau.
